# EUROPÄISCHE PATENTSCHRIFT

(11) **EP 0 686 620 B1**
(45) Veröffentlichungstag und Bekanntmachung des Hinweises auf die Patenterteilung: **14.01.1998**
(21) Anmeldenummer: 95108165.2
(22) Anmeldetag: 29.05.1995
(51) Int. Cl.: C07C 67/04, C07C 69/013, C07C 69/54

(54) **Verfahren zur kontinuierlichen Herstellung von Terpenestern**
Process for the continuous production of terpene esters
Procédé de fabrication continue d'esters terpeniques

(30) Priorität: 06.06.1994 DE 4419686
(43) Veröffentlichungstag der Anmeldung: 13.12.1995
(73) Patentinhaber: Clariant GmbH, 65929 Frankfurt am Main (DE)
(72) Erfinder: Gscheidmeier, Mandred, Dr., D-86456 Gablingen (DE); Gutmann, Rudolf, D-86368 Gersthofen (DE); Wiesmüller, Jakob, D-86462 Stettenhofen (DE); Riedel, Alfred, Dr., D-86391 Stadtbergen (DE)

(56) Entgegenhaltungen:
- DATABASE WPI Week 8318 Derwent Publications Ltd., London, GB; AN 83-42349 & JP-A-58 049 337 (YASUHARA YUSHI KOGY) , 23.März 1983
- DATABASE WPI Week 7423 Derwent Publications Ltd., London, GB; AN 74-42542 & JP-A-49 013 158 (YOSHITOMI PHARM IND LTD) , 5.Februar 1974
- DATABASE WPI Week 7011 Derwent Publications Ltd., London, GB; AN 70-17979 & DD-A-69 586 (KLOSE W.)

## Beschreibung

Die Erfindung bezieht sich auf ein verbessertes Verfahren zur Herstellung von Terpenestern an einem festen Katalysator.

Es ist bekannt, daß man Camphen und Essigsäure im Mol-Verhältnis 1:5,7 bei 60°C kontinuierlich über ein Festbett von Kationaustauscherharz laufen lassen kann und zu guten Esterausbeuten gelangt (vgl. Russisches Patent 102 445).

Weiterhin ist ein Verfahren bekannt, bei welchem Camphen und Essigsäure in äquimolaren Mengen in diskontinuierlicher wie in kontinuierlicher Fahrweise in Gegenwart eines sauren Ionenaustauscherharzes zur Reaktion gebracht werden (vgl. JP 49-13158).

Auch der Einsatz von sulfonsauren Ionenaustauschern aus Styrol-Divinylbenzol-Copolymeren zur Umsetzung von Camphen und Essigsäure in Gegenwart eines aufgewirbelten Katalysators ist bekannt (vgl. DD 69586).

Die bisherige Verwendung von sauren Ionenaustauscherharzen geschah in Laborversuchen, bei denen wegen der kleinen Volumina keine Probleme mit Stoffaustausch und Wärmeabführung bestehen. Geht man zu technischen Produktionsapparaten über, so werden an Produktführung und Temperaturkonstanz wesentlich höhere Anforderungen gestellt als an kleine Versuchsapparaturen:
1. Die Reaktion ist exotherm mit ca. 3 kcal/Mol.
2. Hinsichtlich der Camphen-Umsetzung verläuft sie nur bis zu einer Gleichgewichtseinstellung, die weitestgehend von Temperatur, Mischungsverhältnis und Reinheit der Reaktanden sowie von Qualität und Menge des Katalysators abhängt.
3. Abhängig von der Temperatur, Katalysator-Aktivität und Verweilzeit ist die Bildung von anderen Estern, wie Pseudobornylacetat, Isofenchylacetate oder α-Fenchylacetat, aus Camphen und Tricyclen - einem stets vorhandenen Isomeren - aber auch durch Isomerisation von bereits gebildetem Isobornylacetat. Diese "Nebenester" führen wegen ihres Eigengeruches schon über recht enge Grenzbereiche hinaus zu unerwünschter Abweichung des Gesamtgeruches des Riechstoffes Isobornylacetat.

Aus diesen Gründen ist für die Ausbeute und die Qualität des Zielproduktes ausschlaggebend:
- gleichmäßig gute Wärmeabführung;
- gleichmäßige Produktführung über die ganze Breite und Höhe des Katalysatorbettes;
- möglichst kurze Verweilzeit.

Bei den bisher beschriebenen Reaktionen wird stets nur auf die Gesamtaktivität, in keiner Weise aber auf die geringere Selektivität der Esterbildung geachtet, die beim Wirbelbett vor allem durch uneinheitliche Verweilzeit, beim Festbett zusätzlich durch ungünstigere Temperaturverteilung bedingt ist.

Es wurde nun gefunden, daß diese Nachteile vermieden werden können durch eine Reaktionsdurchführung in einem Katalysator-Schwebebett.

Die Erfindung betrifft somit ein Verfahren zur Herstellung von Terpenestern durch Reaktion von C₁₀H₁₆-Terpenen und einer niedermolekularen Carbonsäure an einem sauren Ionenaustauscher als Katalysator, dadurch gekennzeichnet, daß die Reaktion bei einem Molverhältnis von Terpen zu Carbonsäure von 0,2:1 bis 2:1 und einer Temperatur von 25 bis 60°C durchgeführt wird, wobei die Reaktanten von unten durch den in einem röhrenförmigen Reaktor befindlichen sauren Ionenaustauscher mit einer derartigen Geschwindigkeit geleitet werden, daß der Ionenaustauscher den Reaktor gleichmäßig schwebend erfüllt.

Im erfindungsgemäßen Verfahren wird ein Terpenkohlenwasserstoff mit einer niedermolekularen Carbonsäure umgesetzt.

Geeignete Terpene sind Kohlenwasserstoffe C₁₀H_{16,} insbesondere Camphen, welches bis ca. 20 % Tricyclen enthalten kann.

Geeignete niedermolekulare Carbonsäuren sind gesättigte oder ungesättigte Carbonsäuren bis 1 bis 5 C-Atomen, insbesondere Essigsäure oder Meth/Acrylsäure.

Die Reaktanten werden im Molverhältnis Terpen zu Säure von 0,2:1 bis 2:1, bei gesättigten Säuren vorzugsweise 0,2:1 bis 0,3:1, bei ungesättigten Säuren vorzugsweise 1:1 bis 2:1 eingesetzt.

Die Reaktionstemperatur wird so hoch gewählt, daß die flüssigen Reaktanten ausreichend miteinander reagieren, sie liegt bei 25 bis 60°, vorzugsweise 28 bis 55°C. Der Druck beträgt 1 bis 2, vorzugsweise 1 bis 1,3 bar.

Für das erfindungsgemäße Verfahren wird ein senkrecht stehender, zylindrischer Reaktor verwendet, welcher ein Fassungsvermögen von 200 bis 500, vorzugsweise bis 350 dm³ und ein Verhältnis von Höhe zu Durchmesser von 16:1 bis 19:1 haben sollte. Unten und oben enthält der Reaktor je einen Siebboden mit ausreichend geringer Maschenweite, so daß der körnige Ionenaustauscher weder nach oben noch nach unten entweichen kann. Der Reaktor wird durch spezielle Leitungen mit dem Ionenaustauscher beschickt und entleert. Das als Stützboden ausgelegte Eingangssieb kann zur Vergleichmäßigung der Strömung stellenweise durch Einbauteile, wie Abdeckblenden oder Leitbleche, ergänzt werden.

In den Reaktor wird so viel saurer Ionenaustauscher in Form kleiner Kügelchen eingebracht, daß nach dem Quellen mit den Reaktanten noch ein Reaktor-Leervolumen von 20 bis 40 % besteht. Als Ionenaustauscher eignen sich die handelsüblichen mehr oder minder wasserfeuchten Kationaustauscher, vorzugsweise sulfonsaure Copolymere aus Styrol und Divinylbenzol in Form kleiner Kügelchen, beispielsweie ®Amberlyst 15, ®Bayer K 2611, ®Purolite 175 CT oder ähnliche. Die handelsüblichen Ionenaustauscher, vorzugsweise sulfonsaure Copolymere aus Styrol und Divinylbenzol mit makroporöser Struktur enthalten etwas Wasser und werden deshalb im Reaktor vor Durchführung der Reaktion erst mit Essigsäure/Isobornylmethacrylat gequollen und dann mit zugesetztem Anhydrid der zu verwendenden Säure auf einen Wassergehalt (in der Flüssigkeit) unter 0,1 % "getrocknet".

Von unten wird durch den Siebboden Reaktionsgemisch mit entsprechend gewählter Geschwindigkeit so eingespeist, daß der Ionenaustauscher wegen des mäßigen Dichteunterschiedes gegenüber der Flüssigkeit in leichte Schwebebewegung versetzt wird. Er wird dabei aber nicht einfach im Reaktor nach oben gedrückt oder gewirbelt, sondern er wird je nach Durchströmgeschwindigkeit mehr oder minder über die ganze Reaktorhöhe "auseinandergezogen", so daß er von unten bis oben in einen pseudofluiden Schwebezustand kommt. Um dies zu erreichen, müssen das Höhe/Querschnitt-Verhältnis des Reaktors, das Ionenaustauscher-Volumen und die durchzusetzende Gemischmenge genau aufeinander abgestimmt sein. Gleichzeitig wird die Verweilzeit der Reaktanten so gewählt, daß der gewünschte Umsatz bei der erforderlichen Selektivität erreicht wird. Der ideale Schwebezustand ist dann erreicht, wenn die sich leicht drehende bewegende, aber relativ glatt erscheinende obere Begrenzungsfläche des "gedehnten" Katalysatorbettes bis kurz unter das Austrittssieb des Reaktors reicht. In diesem Betriebszustand ist die Katalysatorbewegung so sanft, daß keinerlei Abrieb beobachtet wird.

Der Wassergehalt des durchzusetzenden Reaktionsgemisches wird möglichst auf einen Wert unter 0,05 % eingestellt, beispielsweise durch Zusatz der erforderlichen Menge des Anhydrids der verwendeten Säure. Die Umsetzung mit dem vorhandenen Wasser kann sowohl im Reaktor selbst, als auch außerhalb des Reaktors erfolgen.

Der vollkontinuierliche Durchsatz wird erst unterbrochen, wenn bei höheren Temperaturen Aktivität und Selektivität nicht mehr ausreichen. Anschließend kann der Ionenaustauscher im Reaktor evtl. erst mit Essigsäure gespült, dann bei erhöhter Temperatur mit einem organischen Lösemittel, beispielsweise Ethanol, von den Ablagerungen an harzartigen Rückständen befreit und dann erneut für die Reaktion eingesetzt werden. Diese Zyklen sind mehrmals möglich, wobei allerdings die Katalysator-Betriebszeit jedesmal etwas kürzer wird.

Bei eventueller Verdichtung des durch Harzbelegung gealterten Katalysators kann durch zusätzliche Maßnahmen, wie ganz kurzes Einblasen von Stickstoff, leichte Pulsationsstöße ins Reaktionsgemisch, kurzzeitige Variation der Strömungsgeschwindigkeit oder dergleichen das gleichmäßige Schweben des Katalysators in der Flüssigkeit wiederhergestellt werden. Auch der Einbau von langsam drehenden Rührern ist vorstellbar.

Bei der Herstellung beispielsweise von Isobornylacetat aus Camphen und Essigsäure läuft die Reaktion nur bis zu einem temperaturabhängigen Gleichgewichtszustand ab; daher ist es sinnvoll, die Reaktion bei 25 bis 27°C zu beginnen und mit allmählich einsetzendem Aktivitätsverlust (durch Bildung von Verharzungs-produkten) die Temperatur langsam zu erhöhen bis ca. 50°C. Eine höhere Temperatur verbessert zwar die Aktivität des Katalysators, führt aber zu Selektivitätsverlust durch verstärkte Bildung von störenden "Nebenestern", wie Isofenchylacetaten und Pseudobornylacetat.

Im technischen Camphen ist stets Tricyclen enthalten, das ebenfalls die Bildung der genannten Nebenester fördert. Daher sollte sein Gehalt im Camphen möglichst unter 18% liegen. Je niedriger dieser Wert liegt, umso höher kann die Temperatur gewählt werden, um eine gleiche Selektivität, aber eine höhere Umsetzungsrate zu erreichen.

Das optimale Mol-Verhältnis Essigsäure : Camphen liegt bei 3,5 : 1 bis 4,5 : 1, da hierbei sowohl die Gleichgewichtsreaktion als auch die Viskosität und Dichte des Reaktionsgemisches zur Einstellung des Katalysator-Schwebezustandes günstig beeinflußt werden. Gleichzeitig reicht der Säure-Überschuß gerade aus, die nicht umgesetzten Terpenkohlenwasserstoffe anschließend nahezu quantitativ azeotrop abdestillieren zu können.

Das erfindungsgemäße Verfahren bringt gegenüber den bekannten Vorgehensweisen zur Herstellung von Terpenestern weitere Verbesserungen:
- hohe spezifische Katalysatorleistung von beispielsweise 2,1 kg Isobornylacetat/dm³ Katalysator/h;
- hohe Selektivität der Reaktion mit Bildung von nur ca. 5,5 % "Nebenestern";
- hoher Umsetzungsgrad von durchschnittlich 80 % des Camphens;
- hohe Katalysator-Gesamtleistung beim 1. Einsatz mit fast 700 kg Isobornylacetat/dm³ Katalysator.

Jeder einzelne dieser Werte läßt sich durch Änderung der Reaktionsbedingungen verbessern unter Inkaufnahme der Verschlechterung bei anderen Werten. (Das nachfolgende Beispiel soll die Erfindung erläutern.)

Bei der Herstellung beispielsweise von Isobornylmethacrylat aus Camphen und Methacrylsäure ist die Bildung von "Nebenestern" etwas weniger kritisch, sodaß die Reaktion bei 45 bis 60°C durchgeführt wird. Da zudem höhere Verweilzeit, also geringere Durchsatzmenge pro Zeit erforderlich ist, kann der pseudofluide Schwebezustand des katalysierenden Ionenaustauscherharzes nur entweder durch einen entsprechend schlankeren Reaktor mit höherer Strömgeschwindigkeit oder mit einem normal dimensionierten Reaktor erreicht werden, wenn gleichzeitig das Reaktionsgemisch durch Umpumpen im Kreislauf geführt wird. Letzteres hat den großen Vorteil, daß der Schwebezustand des Katalysators unabhängig von der Einsatzmenge, also von der Verweilzeit, konstant gehalten werden kann.

Auch Isobornylmethacrylat bildet sich in einer Gleichgewichtsreaktion. Da hierbei nicht ungesetzte Methacrylsäure sehr leicht zur Polymerisation neigt, wird durch das gewählte Molverhältnis Camphen zu Methacrylsäure 2:1 bis 1:1 die Säurekonzentration möglichst klein gehalten. Zusätzlich ist hierbei das Katalysator-Schwebebett von großem Vorteil, da es keine bewegungsfreien Zonen gibt (wie beispielsweise im Festbett-System), die die Polymerisationsgefahr erhöhen.

### Beispiel 1

Ein zylindrischer Reaktor mit einem Leervolumen von 220 dm³, der mit mehreren voneinander unabhängigen Kühlschlangen (jeweils doppelt ausgeführt) ausgestattet war, wurde mit 130 dm³ eines sauren Ionenaustauschers auf Basis Styrol/Divinylbenzol (®Amberlyst 15 dry, Fa. Rohm u. Haas) gefüllt und mit Essigsäure unter Zusatz von Acetanhydrid gequollen. Anschließend wurde von unten in den Reaktor ein Gemisch aus 310 dm³ Camphen (mit durchschnittlich 17,5 % Tricyclen) und 440 dm³ Essigsäure kontinuierlich zudosiert. Bedarfsweise wurde so viel Essigsäureanhydrid zugeführt, daß der Wassergehalt im Rohgemisch unter 0,05% blieb. Die Reaktionstemperatur wurde durch Kühlung zunächst auf 28 bis 29°C gehalten, dann im Laufe von 14 Tagen allmählich angehoben bis 48°C. Nach 7 Tagen wurde zudem der Gesamtdurchsatz auf 700 dm³/h reduziert. Mit fortschreitender Alterung des Katalysators wurde dieser gelegentlich durch kurze Stickstoff-Zufuhr aufgelockert.

Der Camphen-Umsetzungsgrad erreichte im Durchschnitt 80%, die Produkt-Reinheit bezogen auf alle Esterkomponenten 94,4 % Isobornylacetat + 0,3 % Bornylacetat. Bei der anschließenden Destillation wurden insgesamt 89 to Isobornylacetat in Riechstoffqualität mit 99,6 - 99,8 % Gesamtestergehalt gewonnen. Die Katalysator-Leistung betrug somit in 14 Tagen 685 kg Reinprodukt pro dm³ Katalysator.

### Beispiel 2

In einem zylinderischen Reaktionsrohr (Glas) (d = 65 mm, h = 900 mm) mit Doppelmantel und Bodenfritte wurden ca. 1,5 dm³ Isobornylmethacrylat vorgelegt und mit 1,5 dm³ Amberlyst 15 dry (Rohm and Haas) versetzt. Nach Beendigung des Katalysator-Quellvorganges wurde von unten so lange Reaktionsgemisch, bestehend aus technisch reinem Camphen und Methacrylsäure im Molverhältnis 1,5:1 plus 0.02 % Phenothiazin als Stabilisator, zugesetzt, bis eine Kreislaufführung des Gemisches durch den Katalysator möglich wurde. Die Umlaufgeschwindigkeit wurde dann so eingestellt, daß das Katalysatorbett auf das 1,5- bis 2-fache Volumen gedehnt wurde. Anschließend wurde der Reaktor durch Temperieren über einen in den Kreislauf eingebauten Wärmetauscher und mittels Warmwasser im Doppelmantel auf eine Tempertur von 45 bis 50°C gebracht, ehe mit der kontinuierlichen Zuspeisung von weiterem Reaktionsgemisch begonnen wurde. Die Reaktorinnentemperatur wurde dann auf 50°C konstant gehalten. Bei einer Zuspeisung von 1 dm³ Reaktionsgemisch pro Stunde wurde die gleiche Menge durch einen hochgezogenen Überlauf aus dem Kreislauf-System abgenommen als Rohprodukt zur nachfolgenden Destillation; es enthieltca. 30 % TerpenKohlenwasserstoffe, ca. 65 % Ester und ca. 5 % Restsäure.

Im Laufe von ca. 50 h war nur geringer Aktivitätsverlust zu beobachten, der durch Temperaturerhöhung ausgeglichen werden konnte. Trübung durch Polymerbildung war nicht eingetreten. Auch anschließend war das Rohprodukt sehr stabil und konnte durch Vakuumdestillation aufgetrennt werden zu Camphenüberschuß-Fraktion (mit weniger Säure) und zu Ester-Fraktion mit 98-99 % Estern (davon ca. 88 % Isobornylmethacrylat).

Bei Einsatz von reinerem Camphen nimmt der Gehalt an Isobornylmethacrylat je nach dem Gehalt an restlichen, im Camphen stets enthaltenen Tricyclen zu bis ca. 95 %.

## Patentansprüche

1. Verfahren zur Herstellung von Terpenestern durch Reaktion von C₁₀H₁₆-Terpenen und einer niedermolekularen Carbonsäure an einem sauren Ionenaustauscher als Katalysator, dadurch gekennzeichnet, daß die Reaktion bei einem Molverhältnis von Terpen zu Carbonsäure von 0,2:1 bis 2:1 und einer Temperatur von 25 bis 60°C durchgeführt wird, wobei die Reaktanten von unten durch den in einem säulenförmigen Reaktor befindlichen sauren Ionenaustauscher mit einer derartigen Geschwindigkeit geleitet werden, daß der Ionenaustauscher den Reaktor gleichmäßig schwebend erfüllt.

2. Verfahren nach Anspruch 1, dadurch gekennzeichnet, daß das Terpen ein Tricyclen enthaltendes Camphen ist.

## Claims

1. A process for preparing terpene esters by reaction of C₁₀H₁₆-terpenes and a low molecular weight carboxylic acid over an acid ion exchanger as catalyst, which comprises carrying out the reaction at a molar ratio of terpene to carboxylic acid of from 0.2:1 to 2:1 and a temperature of from 25 to 60°C, with the reactants being passed from below through the acid ion exchanger located in a column-shaped reactor at such a velocity that the ion exchanger is suspended to uniformly fill the reactor.

2. The process as claimed in claim 1, wherein the terpene is a camphene containing tricyclene.

## Revendications

1. Procédé de préparation d'esters terpéniques par réaction de terpènes en C₁₀H₁₆ et d'un acide carboxylique de bas poids moléculaire, sur un échangeur d'ions acide comme catalyseur, caractérisé en ce que l'on met en oeuvre la réaction avec un rapport molaire du terpène à l'acide carboxylique de 0,2:1 à 2:1 et une température de 25 °C à 60 °C, en introduisant les réactants par le bas, à travers l'échangeur d'ions acide se trouvant dans un réacteur cylindrique, à une vitesse telle, que l'échangeur d'ions remplisse uniformément, en flottant, le réacteur.

2. Procédé selon la revendication 1, caractérisé en ce que le terpène est un camphène contenant du tricyclène.
